(19) Europäisches Patentamt
European Patent Office
Office européen des brevets

(11) Veröffentlichungsnummer: **0 100 509**
**B1**

(12) # EUROPÄISCHE PATENTSCHRIFT

(45) Veröffentlichungstag der Patentschrift: **21.05.86**

(51) Int. Cl.⁴: **H 01 B 1/12**

(21) Anmeldenummer: **83107276.4**

(22) Anmeldetag: **25.07.83**

(54) **Verfahren zur Herstellung elektrisch leitfähiger Polyarylen-Verbindungen mit elektronenanziehenden Seitengruppen und deren Verwendung in der Elektrotechnik und antistatischen Ausrüstung von Kunststoffen.**

(30) Priorität: **03.08.82 DE 3228880**

(43) Veröffentlichungstag der Anmeldung: **15.02.84 Patentblatt 84/7**

(45) Bekanntmachung des Hinweises auf die Patenterteilung: **21.05.86 Patentblatt 86/21**

(84) Benannte Vertragsstaaten: **BE DE FR GB NL**

(56) Entgegenhaltungen:
**EP-A-0 029 945**
**EP-A-0 062 837**

(73) Patentinhaber: **BASF Aktiengesellschaft, Carl-Bosch-Strasse 38, D-6700 Ludwigshafen (DE)**

(72) Erfinder: **Naarmann, Herbert, Dr., Haardtblick 15, D-6719 Wattenheim (DE)**
Erfinder: **Simak, Petr, Dr., Philipp-Scheidemann-Strasse 17, D-6700 Ludwigshafen (DE)**
Erfinder: **Koehler, Gernot, Dr., Berner Weg 32, D-6700 Ludwigshafen (DE)**

**EP 0 100 509 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft ein Verfahren zur Herstellung von elektrisch leitfähigen polymeren Systemen mit elektrischen Leitfähigkeitswerten größer als 10-4 S/cm aus Polyarylen-Verbindungen durch Behandeln der Polyarylen-Verbindungen unter Ausschluß von Wasserfeuchtigkeit und von Sauerstoff mit 0.03 bis 0,9 Molprozent, bezogen auf die eingesetzte Polyarylen-Verbindung, einer starken Lewis-Säure mit $pk_s$-Werten von $-10$ bis $+4$ oder eines Alkalimetalls.

Durch die Dotierung mit den starken Lewis-Säuren bzw. den Alkalimetallen wird eine Erhöhung der elektrischen Leitfähigkeit der Polyarylen-Verbindungen bewirkt.

Aus der DE-OS 29 47 797 ist es bereits bekannt, die elektrische Leitfähigkeit von Polyen-Verbindungen, die mindestens einmal das Kettenglied der Formel:

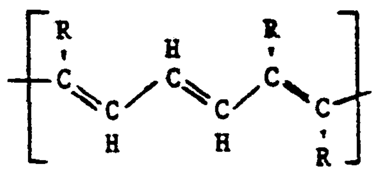,

in der R ein Wasserstoffatom oder eine Methylgruppe bedeutet, und insgesamt mindestens sieben aliphatische Doppelbindungen enthalten, zu erhöhen, indem man die Polyen-Verbindungen unter Ausschluß von Wasserfeuchtigkeit und von Sauerstoff mit 0,03 bis 0,9 Molprozent einer starken Lewis-Säure wie $AsF_5$, $SbF_5$, $FSO_3H$, $HClO_4$, $NO^+AsF_6^-$ oder eines Alkalimetalls behandelt. Die erhaltenen leitfähigen Polyen-Verbindungen sind außerordentlich empfindlich gegenüber der Einwirkung von Feuchtigkeit und Luftsauerstoff.

Des weiteren ist es aus der DE-OS 29 47 796 sowie Polymer, Vol. 20 (1979), Seiten 1441 bis 1443, bekannt, Poly(phenylenvinylene) der Formel (Ph-CH = CH) zur Erhöhung der elektrischen Leitfähigkeit mit Alkalimetallen bzw. starken Lewis-Säuren, wie $AsF_5$, zu dotieren. Doch auch diese elektrisch leitfähigen Systeme sind gegenüber Sauerstoff und/oder Wasser instabil und verlieren rasch ihre Leitfähigkeit, wenn man sie bei Raumtemperatur der Luft aussetzt (vgl. den oben zitierten Artikel in "Polymer").

In der DE-OS 31 14 342 ist bereits vorgeschlagen worden, elektrisch leitfähige Polyen-Systeme mit verbesserter Hydrolysebeständigkeit und erhöhter Stabilität gegenüber Sauerstoff herzustellen, indem man Polyen-Verbindungen mit rein aliphatischen Polymer-Hauptketten, die durch Nitril-, Säureamid- oder Phenylgruppen substituiert sind, mit Alkalimetallen oder starken Lewis-Säuren dotiert.

Der Erfindung liegt die Aufgabe zugrunde, elektrisch leitfähige Polyarylen-Verbindungen zu schaffen, die in einfacher Weise durch Dotierung entsprechend dem Stand der Technik herstellbar sind, die aber eine geringere Hydrolysetendenz, bessere Verarbeitbarkeit sowie zusätzlich eine erhöhte Stabilität gegenüber Luftsauerstoff und Wärme aufweisen.

Diese Aufgabe wird erfindungsgemäß dadurch gelöst, daß man solche Polyarylen-Verbindungen mit elektronenanziehenden oder elektronenabgebenden Komplexierungsmitteln dotiert, die mindestens einmal das Kettenglied der Formel:

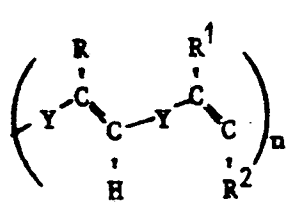

enthalten, wobei n jeweils größer als 1, bevorzugt 5 bis 50, ist, R eine Nitril-, Säureamid-, Carboxyl- oder Estergruppe und $R^1$ und $R^2$ ein Wasserstoffatom oder eine Nitril-, Säureamid-, Carboxyl- oder Estergruppe darstellen mit der Maßgabe, daß stets der eine der Reste $R^1$ oder $R^2$ ein Wasserstoffatom und der andere dieser beiden Reste eine Nitril-, Säureamid-, Carboxyl- oder Estergruppe ist und Y einen aromatischen Rest, vorzugsweise Phenylen, bedeutet.

Unter elektrisch leitfähigen polymeren Systemen mit elektrischen Leitfähigkeitswerten größer als $10^{-4}$ S/cm werden Substanzen verstanden, die nach der Meßmethode von F. Beck, Berichte der Bunsengesellschaft 68 (1964), Seiten 558 bis 567 eine elektrische Leitfähigkeit von mehr als $10^{-4}$ S/cm aufweisen.

Die erfindungsgemäß einzusetzenden Polyarylen-Verbindungen werden nach den an sich bekannten Methoden unter Ausschluß von Wasserfeuchtigkeit und von Sauerstoff mit 0,03 bis 0,9 Molprozent, bezogen auf die eingesetzte Polyarylen-Verbindung, insbesondere einer starken Lewis-Säure mit $pk_s$-Werten von $-10$ bis $+4$ oder eines Alkalimetalls als Komplexierungsmittel behandelt.

Als Lewis-Säuren mit $pk_s$-Werten von $-10$ bis $+4$ (n-Komplexierungsmittel) eignen sich z.B. die Verbindungen

AsF$_5$, SbF$_5$, HClO$_4$, FSO$_3$H, C10$_2$, NO+SbF$_6^-$, NO$_2$+SbF$_6^-$, NO+AsF$_6^-$, NO$_2$+AsF6-, NO+PF$_6^-$, NO$_2$+PF$_6^-$, NO+B F$_4^-$, NO$_2$+BF$_4^-$, NO+C10$_4^-$, (CF3)$_2$SO$_4$ 2,4,6-Trinitrophenol, 2,4,6-Trinitrobenzolsulfonsäure oder 2,4,6-Trinitrobenzoesäure.

Als Alkalimetalle (p-Komplexierungsmittel) verwendet man z.B. Natrium oder Kalium, die auch in Form von Lösungen, z.B. in Naphthalin oder α-Methylstyrol, eingesetzt werden können.

Die Behandlung der Polyarylen-Verbindungen mit den Lewis-Säuren oder den Alkalimetallen führt man bei Temperaturen von −70 bis 150°C, vorzugsweise −10 bis 100°C, insbesondere 0 bis 30-C durch. Dabei wendet man die Lewis-Säuren oder Alkalimetalle, die mit den Polyarylenen unter Komplexbildung die elektrisch leitfähigen Systeme ergeben, in Mengen von 0,03 bis 0,9, vorzugsweise von 0,1 bis 0,5 Molprozent, bezogen auf die Polyarylen-Verbindungen, an.

Das Einarbeiten der Komplexierungsmittel erfolgt unter Ausschluß von Feuchtigkeit (Wasser) sowie Sauerstoff (Luft), es wird daher vorzugsweise unter Edelgasatmosphäre (Argon) gearbeitet. Gegebenen alls werden als Hilfsflüssigkeiten nicht-wäßrige Lösungsmittel, die unter den Verfahrensbedingungen nicht mit den Komplexierungsmitteln reagieren, wie Methylenchlorid, Tetrahydrofuran, Dimethoxyglykol, Nitromethan, Naphthalin oder α-Methylstyrol, eingesetzt. Die meist tieffarbigen elektrisch leitfähigen Polyarylene werden bei dem erfindungsgemäßen Verfahren in wenigen Sekunden bis Minuten gebildet. Gegebenenfalls verwendete Lösungsmittel werden zweckmäßig bei Temperaturen unter 100°C im Vakuum abgezogen.

In dem erfindungsgemäßen Verfahren werden Polyarylen-Verbindungen eingesetzt, die mindestens einmal das Kettenglied der Formel:

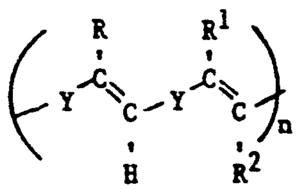

enthalten, wobei n größer als 1, bevorzugt 5 bis 50, ist, R eine Nitril-, Säureamid-, Carboxyl- oder Estergruppe und R eine Nitril-, Säureamid-, Carboxyl- oder Estergruppe und R$^1$ und R$^2$ ein Wasserstoffatom oder eine Nitril-, Säureamid-, Carboxyl- oder Estergruppe darstellen mit der Maßgabe, daß stets der eine der Reste R$^1$ oder R$^2$ ein Wasserstoffatom und der andere dieser beiden Reste eine Nitril-, Säureamid-, Carboxyl- oder Estergruppe ist, und Y einen aromatischen Rest, vorzugsweise Phenylen, bedeutet. Die erfindungsgemäß zu verwendenden Polyarylen-Verbindungen sind an sich bekannt und entsprechend der Literaturstelle DE-OS 16 18 733 herstellbar. Zweckmäßigerweise werden aromatische Dialdehyde, z.B. Terephthal-Dialdehyd, z.B. mit Bernsteinsäuredinitril und/oder 1,2-Diphenyl-äthan im Molverhältnis 1: 1 in Gegenwart von Basen wie Natriumamid oder Kalium-tert.-butylat in Toluol bis zu acht Stunden auf Siedetemperatur erhitzt.

Für das erfindungsgemäße Verfahren sind die Polyarylen-Verbindungen der nachfolgenden Formeln I bis X besonders geeignet.

I

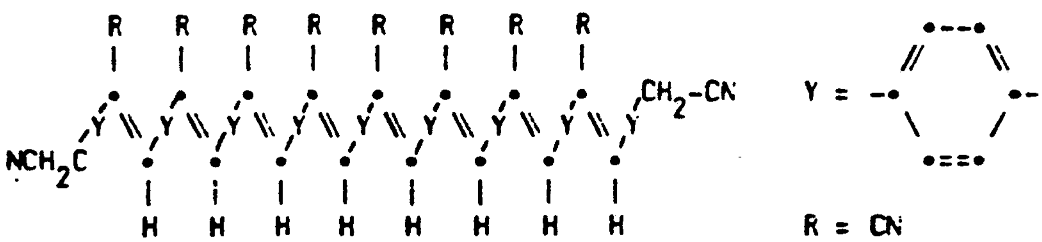

R = CN

II

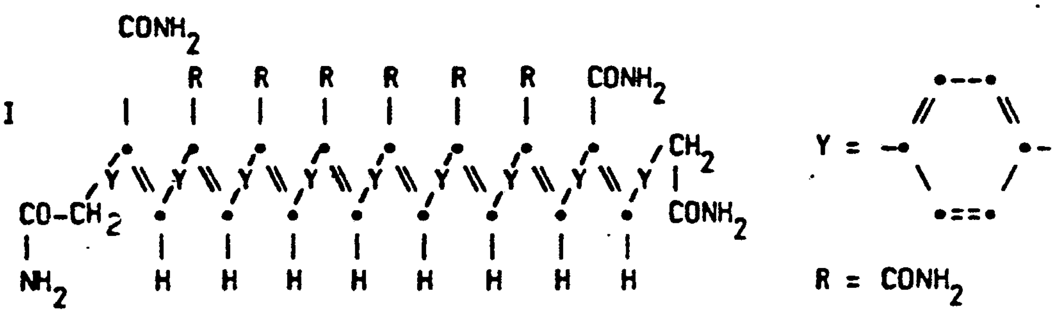

R = CONH$_2$

III

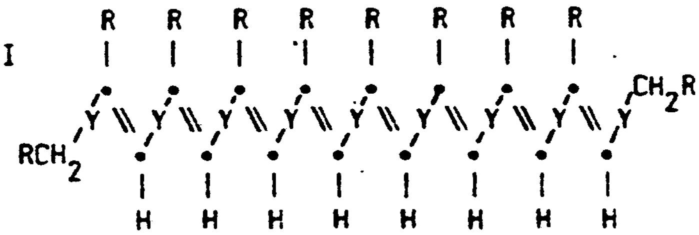

Y =

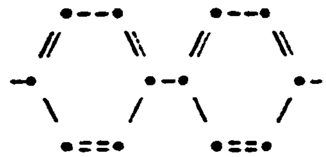

R = CN

IV

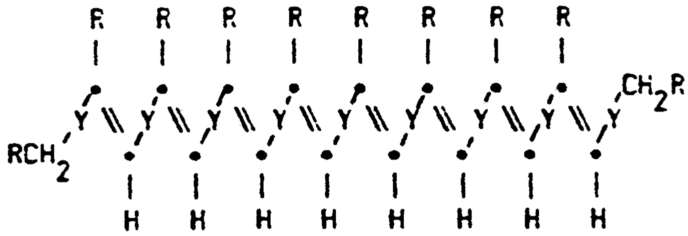

Y =

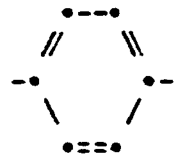

R = COOH

V

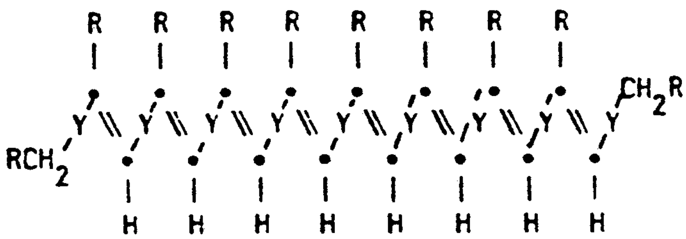

Y =

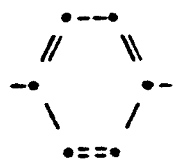

R = $COOCH_3$

VI

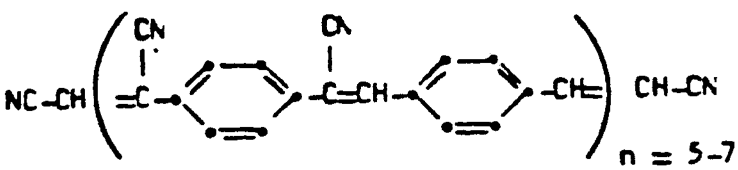

n = 5-7

VII

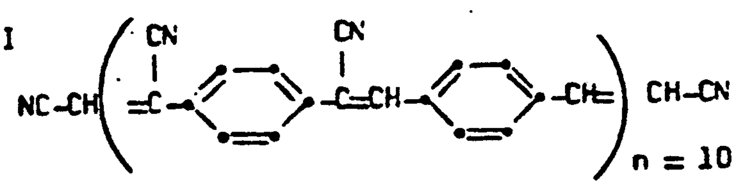

n = 10-15

VIII

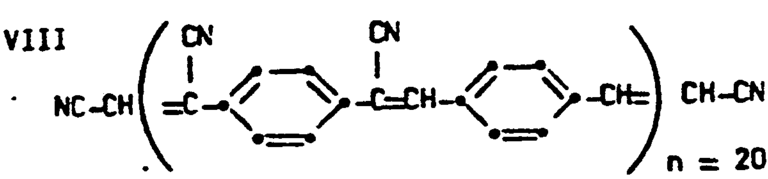

n = 20-30

IX

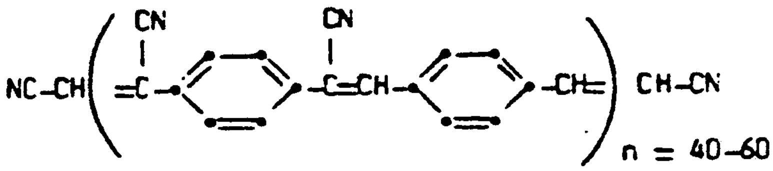

X

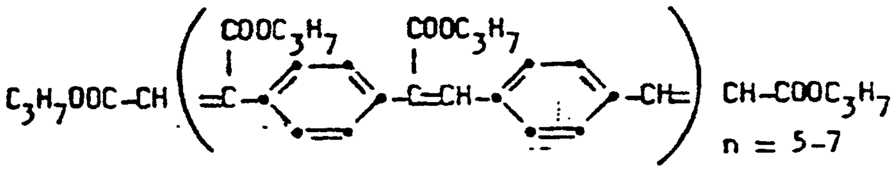

Die erfindungsgemäß hergestellten elektrisch leitfähigen Polyarylene sind zur antistatischen Ausrüstung von Kunststoffen, zur Herstellung von Sonnenzellen, zur Umwandlung und Fixierung von elektromagnetischen Strahlen sowie zur Herstellung elektrischer und magnetischer Schalter und elektrischer Speicher verwendbar.

Die in den folgenden Beispielen genannten Teile sind Gewichtsteile.

**Beispiele 1 bis 12**

Zu 10 Teilen der jeweiligen Poyarylen-Verbindung wurde unter Argon bei Raumtemperatur und unter Ausschluß von Wasserfeuchtigkeit und Luftsauerstoff ein n- oder p-Komplexierungsmittel gegeben. Die Komplexbildung trat sofort ein. Man erhielt tiefblauschwarze kristalline Verbindungen, die beim Messen in einer Leitfähigkeitsmeßzelle eine Leitfähigkeit von $>10^{-4}$ S/cm aufwiesen. Die Leitfähigkeit der Ausgangspolyarylene betrug ca. $10^{-10}$ S/cm. Die in den einzelnen Beispielen eingesetzten Polyarylene und Komplexierungsmittel sowie die elektrischen Leitfähigkeiten der erhaltenen Produkte sind in der Tabelle wiedergegeben. Die Polyarylene sind dabei mit der weiter oben in der Beschreibung benutzten Bezifferung gekennzeichnet.

| Beispiel | Polyarylen Art und Menge | Ausgangsleitfähigkeit bei 25° C [S/cm] | Komplexierungsmittel Menge [Teile] und Art | Leitfähigkeit nach dem Dotieren bei 25° C [S/cm] |
|---|---|---|---|---|
| 1 | I<br>10 Teile | $1{,}0 \cdot 10^{-10}$ | 15 $SbF_5$<br>ca 0,1 Mol % | $4{,}2 \cdot 10^{-3}$ |
| 2 | II<br>10 Teile | $1{,}4 \cdot 10^{-10}$ | 15 $SbF_5$<br>ca 0,1 Mol % | $2{,}5 \cdot 10^{-3}$ |
| 3 | III<br>10 Teile | $1{,}4 \cdot 10^{-10}$ | 18 $NO^+SbF_6$<br>ca 0,1 Mol % | $2{,}8 \cdot 10^{-3}$ |
| 4 | IV<br>10 Teile | $1{,}4 \cdot 10^{-10}$ | 20 $NO_2^+SbF_6$<br>ca 0,1 Mol % | $3{,}6 \cdot 10^{-3}$ |
| 5 | V<br>10 Teile | $1{,}4 \cdot 10^{-10}$ | 6 K<br>ca 0,2 Mol % | $2{,}0 \cdot 10^{-2}$ |
| 6 | VI<br>10 Teile | $3{,}5 \cdot 10^{-10}$ | 20 $SbF_5$<br>ca 0,2 Mol % | $6{,}5 \cdot 10^{-2}$ |
| 7 | VII<br>10 Teile | $2{,}4 \cdot 10^{-10}$ | 15 $AsF_5$<br>ca 0,1 Mol % | $8{,}5 \cdot 10^{-1}$ |
| 8 | VIII<br>10 Teile | $1{,}0 \cdot 10^{-10}$ | 21 $SbF_5$<br>ca 0,2 Mol % | $5{,}0 \cdot 10^{-2}$ |
| 9 | IX<br>10 Teile | $1{,}0 \cdot 10^{-10}$ | 15 $AsF_5$<br>ca 0,1 Mol % | $0{,}5 \cdot 10^{-1}$ |
| 10 | I<br>10 Teile | $1{,}0 \cdot 10^{-10}$ | 5 Na<br>ca 0,2 Mol % | $2{,}5 \cdot 10^{-3}$ |
| 11 | I<br>10 Teile | $1{,}0 \cdot 10^{-10}$ | 5 $AsF_5$<br>ca 0,05 Mol % | $3{,}5 \cdot 10^{-2}$ |
| 12 | I<br>10 Teile | $1{,}0 \cdot 10^{-10}$ | 5 $NO_2^+PF_6^-$<br>ca 0,05 Mol % | $2{,}0 \cdot 10^{-1}$ |

**Patentansprüche**

1. Verfahren zur Herstellung von elektrisch leitfähigen polymeren Systemen mit elektrischen Leitfähigkeitswerten größer als $10^{-4}$ S/cm aus Polyarylen-Verbindungen durch Behandeln der Polyarylen-Verbindungen unter Ausschluß von Wasserfeuchtigkeit und von Sauerstoff mit 0,03 bis 0,9 Molprozent, bezogen auf die eingesetzte Polyarylen-Verbindung, eines elektronenanziehenden oder elektronenabgebenden Komplexierungsmittels, dadurch gekennzeichnet, daß man Polyarylen-Verbindungen einsetzt, die mindestens einmal das Kettenglied der Formel:

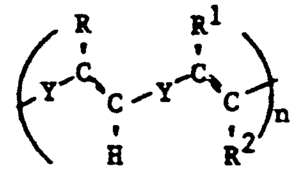

enthalten, wobei n jeweils größer als 1 ist, R eine Nitril-, Säureamid-, Carboxyl- oder Estergruppe und $R^1$ und $R^2$ ein Wasserstoffatom oder eine Nitril-, Säureamid-, Carboxyl- oder Estergruppe darstellen mit der Maßgabe, daß stets der eine der Reste $R^1$ oder $R^2$ ein Wasserstoffatom und der andere dieser beiden Reste eine Nitril-, Säureamid-, Carboxyl- oder Estergruppe ist, und Y einen aromatischen Rest bedeutet.

2. Verfahren nach Anspruch 1, dadurch gekennzeichnet, daß n 5 bis 50 ist.

3. Verfahren nach Anspruch 1 oder 2, dadurch gekennzeichnet, daß Y ein Phenylenrest ist.

4. Verfahren nach einem der Ansprüche 1 bis 3, dadurch gekennzeichnet, daß als elektronenanziehendes Komplexierungsmittel eine starke Lewis-Säure mit $pk_s$-Werten von -10 bis +4 eingesetzt wird.

5. Verfahren nach einem der Ansprüche 1 bis 3, dadurch gekennzeichnet, daß als elektronenabgebendes Komplexierungsmittel ein Alkalimetall, insbesondere Natrium oder Kalium, eingesetzt wird.

6. Verfahren nach einem der Ansprüche 1 bis 5, dadurch gekennzeichnet, daß das Komplexierungsmittel in Mengen von 0,1 bis 0,5 Mol-%, bezogen auf die eingesetzte Polyarylen-Verbindung, verwendet wird.

7. Verwendung der nach den Ansprüchen 1 bis 6 hergestellten elektrisch leitfähigen polymeren Systeme in der Elektrotechnik zur Herstellung von Sonnenzellen, zur Umwandlung und Fixierung von elektromagnetischen Strahlen und zur Herstellung elektrischer und magnetischer Schalter und elektrischer Speicher.

8. Verwendung der nach den Ansprüchen 1 bis 6 hergestellten elektrisch leitfähigen polymeren Systeme zur antistatischen Ausrüstung von Kunststoffen.

**Revendications**

1. Procédé de préparation de systèmes polymères conducteurs de l'électricité avec des conductivités électriques supérieures à $10^{-4}$ S/cm à partir de composés polyaryléniques par le traitement de ces composés polyaryléniques, à l'abri de l'humidité d'eau et de oxygène, avec 0,03 à 0,9 % molaire par rapport au composé polyarylénique employé d'un agent complexant attirant les électrons ou cédant des électrons, caractérisé en ce que l'on utilise des composés polyaryléniques comprenant au moins une fois un maillon de chaîne de la formule

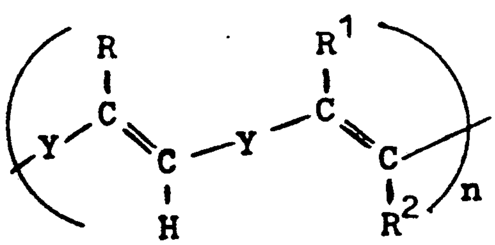,

dans laquelle n est supérieur à 1, R désigne un groupe nitrile, amide d'acide, carboxyle ou ester et $R^1$ et $R^2$ désignent chacun un atome d'hydrogène ou un groupe nitrile, amide d'acide, carboxyle ou ester, avec la condition que l'un des substituants $R^1$ et $R^2$ est toujours un atome d'hydrogène et l'autre des deux un groupe nitrile, amide d'acide, carboxyle ou ester, et Y représente un groupe aromatique.

2. Procédé suivant la revendication 1, caractérisé en ce que n vaut de 5 à 50.

3. Procédé suivant la revendication 1 ou 2, caractérisé en ce que Y est un groupe phénylène.

4. Procédé suivant l'une des revendications 1 à 3, caractérisé en ce que l'on emploie comme agent complexant attirant les électrons un acide de Lewis fort avec un $pk_a$ entre $-10$ et $+4$.

5. Procédé suivant l'une des revendications 1 à 3, caractérisé en ce que l'on emploie comme agent complexant cédant des électrons un métal alcalin, en particulier du sodium et du potassium.

6. Procédé suivant l'une des revendications 1 à 5, caractérisé en ce que l'agent complexant est mis en oeuvre à raison de 0,1 à 0,5 % molaire par rapport au composé polyarylénique utilise.

7. Utilisation des systèmes polymères conducteurs de l'électricité, préparés selon les revendications 1 à 6, dans le domaine électro-technique pour la fabrication de cellules solaires, pour la transformation et la fixation de radiations électro-magnétiques et pour la fabrication de commutateurs électriques et magnétiques et d' accumulateurs électriques.

8. Utilisation des systèmes polymères conducteurs de l'électricité, préparés selon les revendications 1 à 6, pour l'apprêtage antistatique de matières plastiques.

## Claims

1. A process for the preparation of an electrically conductive polymeric system, having an electrical conductivity greater than $10^{-4}$ S/cm, from a polyarylene compound by treating the said compound, in the absence of moisture and oxygen, with from 0.03 to 0.9 mole per cent, based on the polyarylene compound employed, of an electronattracting or electron-donating complexing agent, wherein the polyarylene compound employed contains one or more chain members of the formula

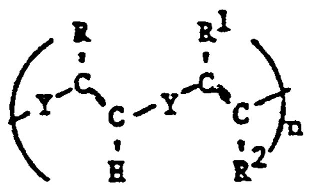

where n is greater than 1, R is a nitrile, acid amide, carboxyl or ester group, $R^1$ and $R^2$ are each hydrogen or a nitrile, acid amide, carboxyl or ester group, with the proviso that in each case one of the radicals $R^1$ and $R^2$ is hydrogen and the other is a nitrile, acid amide, carboxyl or ester group, and Y is an aromatic radical.

2. A process as claimed in claim 1, wherein n is from 5 to 50.

3. A process as claimed in claim 1 or 2, wherein Y is phenylene.

4. A process as claimed in any of claims 1to 3, wherein a strong Lewis acid having a pKs of from -10 to $+4$ is used as the electron-attracting complexing agent.

5. A process as claimed in any of claims 1 to 3, wherein an alkali metal, in particular sodium or potassium, is used as the electron-donating complexing agent.

6. A process as claimed in any of claims 1 to 5, wherein the complexing agent is used in an amount of from 0.1 to 0.5 mole %, based on the polyarylene compound employed.

7. The use of an electrically conductive polymeric system, prepared as claimed in claims 1 to 6, in electrical engineering for the production of solar cells, for the conversion and fixation of electromagnetic radiation, and for the production of electrical and magnetic switches and electrical storage devices.

8. The use of an electrically conductive polymeric system, prepared as claimed in claims 1 to 6, for the antistatic treatment of plastics.